# EUROPEAN PATENT APPLICATION

(11) **EP 3 187 136 A1**
(43) Date of publication of application: **05.07.2017**
(21) Application number: 15003679.6
(22) Date of filing: 29.12.2015
(51) Int. Cl.: A61B 18/12

(54) **BLOOD VESSEL SEALING DEVICE**

(71) Applicant: Valtronic Technologies (Holding) SA, 1343 Les Charbonnières (CH)
(72) Inventor: Lepple-Wienhues, Albrecht, 25300 Pontarlier (FR)
(74) Representative: Helbig, Christian

(57) **Abstract**

The invention relates to a blood vessel sealing device comprising a clamp which has a pair of jaws that can move relative to each other for inserting and crimping a blood vessel, said jaws containing high-frequency (HF) jaw electrodes (12a, 12b), and comprising an electrical HF power supply circuit (8) including an HF generator (10) and the HF jaw electrodes.The blood vessel sealing device according to the invention further comprises an impedance control device (9) for continuously measuring and maintaining constant the impedance of the HF power supply circuit in the course of the respective sealing operation. For this purpose, the HF generator includes a variable impedance HF resonant circuit with a capacitor unit (13) and a coil unit (14), and comprising the jaw electrodes, wherein the inductance of the coil unit and/or the ohmic resistance of the HF resonant circuit is/are variably adjustable and/or wherein the capacitance of the capacitor unit is variably adjustable and the capacitor unit comprises an electrically controllable capacitance diode or at least one movable capacitance-altering capacitor electrode arranged in the clamp.

A blood vessel sealing device of this type can be used, for example, for sealing of vessels for blood donation bags.

## Description

The invention relates to a surgical blood vessel sealing device comprising a clamp having a pair of jaws that can move relative to each other for compressing a blood vessel and contain high-frequency (HF), i.e. radio frequency (RF), electrodes, and further comprising an electrical HF power supply circuit which includes an HF generator and the HF electrodes of the jaws.

Blood vessel sealing devices of this type are used in various ways in surgery and can contain further functions like cutters. A design as a handheld device with a handheld device body is preferred. The conventional handheld devices typically have a cabled connection to an electrical power supply source, such as a stationary or shoulder-strapped battery pack.

It is known that those sealer clamps undergo an impedance change when the distance between the electrodes changes and the tissue is heated during sealing. This impedance change can detune resonance and cause unwanted reflections, thus adversely affecting the efficiency of the sealing process and the quality of the seal.

It is the technical problem underlying the invention to provide a blood vessel sealing device of the type mentioned in the introduction, which enables a reliable, process-safe sealing of vessels. High energy efficiency is required in order to construct a device that is cordless and provides low weight and high user comfort. The device is cordless and contains the clamp, the RF circuitry and the battery in a handheld unit.

The invention solves this problem by providing a blood vessel sealing device having the features of claim 1. In this device, the HF power supply circuit contains a high-frequency generator with a variable-impedance HF resonant circuit, for which the capacitance of the associated capacitor unit and/or the inductance of the associated coil unit and/or the ohmic resistance of the HF resonant circuit are/is variably adjustable. Furthermore, the device according to the invention comprises an impedance control device configured to act towards maintaining a constant impedance of the HF power supply circuit in the course of the respective sealing operation. To this end the impedance of the HF power supply circuit may be measured, preferably in a continuous manner during the sealing operation. In this option, the impedance control device can be designed additionally as an impedance measurement device. In corresponding embodiments of the invention the capacitor unit comprises an electrically controllable capacitance diode or at least one movable capacitance-altering capacitor electrode which is arranged close to the clamp or in the clamp, preferably in the jaw part of the clamp, to achieve the variable capacitance of the capacitor unit. When using a capacitor diode, the capacitance of this diode can be controlled electronically, and the diode can, in the present case, be designed specifically such that the change in capacitance thereof is counteracted in a compensating manner by that of the HF jaw electrodes.

As a result of this construction, the blood vessel sealing device according to the invention is capable of maintaining essentially constant the impedance of the HF power supply circuit, which changes owing to the movement of the jaws and the alteration of the vessel material between the jaws due to heating during the sealing operation, without having to change the frequency of the high-frequency radiation used for the sealing operation in order to do so. This is of great advantage for the reason, among others, that, in the environments in which such vessel sealing devices are typically used, high-frequency fields with frequencies other than quite specific, prespecified frequencies, such as the frequency value for the high frequency used for the sealing operation, are generally not desired or even not permitted. Further, the device of the invention can advantageously be realized as a mobile, lightweight, handheld device, where its total weight may be less than 450g and preferably less than 300g. For keeping the weight at a minimum it may be preferred to use a lightweight battery pack, such as of the Li-ion polymer type.

In an enhancement of the invention, the capacitor unit includes a movable capacitance-altering dielectric element. Alternatively or additionally, the coil unit has a movable inductance-altering element, such as, for example, a ferrite element. This also enables a desired adjustment in the impedance of the HF power supply circuit to be accomplished during the sealing operation in a simply designed manner.

In another embodiment, the movable capacitance-altering capacitor electrode is arranged in the clamp in such a way that a closing movement of the jaw electrodes is compensated for by an opposite movement of capacitor electrodes of the capacitor unit connected in series or in parallel to the HF jaw electrodes so that the capacitance of the capacitor unit changes oppositely to the capacitance of the HF jaw electrodes during a closing movement of the jaw electrodes. In this way, it is possible in a simple manner to achieve a capacitance compensation and consequently to substantially maintain a constant capacitance by proper parallel or serial electrical connection of these two capacitances in the circuit.

In an advantageous embodiment of the invention the movable capacitance-altering capacitor electrode is mechanically coupled to one of the jaws containing the jaw electrodes. This can simplify the device and provides a direct coupling of the capacitor electrode movement to the jaw movement.

More generally, in cases where movement of a movable element of the variable impedance HF resonant circuit is coupled to the movement of the clamp, i.e. to at least one of its jaws, the impedance control circuit may just be realized by this coupling, such as a mechanical coupling or electrical or magnetic coupling or hydraulic or pneumatic coupling, without needing additional control elements.

In an embodiment of the invention, the required change of capacity and/or inductance of the variable impedance HF resonant circuit can be realized by switching between two or more discrete capacitors and/or inductors/coils alternatively to a continuous change of capacitance or inductance. Such switching may be mechanically or in any other conventional manner coupled to the movement of the clamp jaws during the sealing process.

In an enhancement of the invention, the blood vessel sealing device has a same, common electrode which forms one of the capacitor electrodes of the capacitor unit and one of the jaw electrodes. This again allows to simplify the arrangement while maintaining superior sealing characteristics. In a further development, said same electrode forms an intermediate electrode positioned between two outer electrodes forming a counter electrode of the capacitor unit and the other jaw electrode, respectively. In this case, a capacitor of the capacitor unit may contain one of the outer electrodes and the intermediate electrode, and the other outer electrode forms the second jaw electrode.

In a further refinement, said other jaw electrode and said counter electrode are coupled electrically to a same potential so that a capacitance of the jaw electrodes and a capacitance of the capacitor unit are connected in parallel. In an alternative refinement, the intermediate electrode, the other jaw electrode, and the counter electrode are arranged so that a capacitance of the jaw electrodes and a capacitance of the capacitor unit are connected in series.

An additional capacitor electrode of the capacitor unit can be provided separately by the intermediate electrode or by an additional electrode, which is preferably arranged between the intermediate electrode and the capacitor electrode.

In an enhancement of the invention, the intermediate electrode of the device represents the movable capacitance-altering capacitor electrode. The movable capacitance-altering capacitor electrode according to enhancements of this type can be arranged movably between the outer HF jaw electrode and the other capacitor electrode.

Advantageously, in an enhancement of the invention, the intermediate HF jaw electrode is coupled to one of the jaws of the blood vessel sealing unit.

As a person skilled in the art will realize, the enhancements presented above also apply to devices that do not necessarily have jaws in a narrow sense, but rather have other means that are suited for bearing the electrodes for the vessel sealing device according to the invention and should be understood to be covered by the expression jaw as used herein. In addition, it is to be understood that the enhancements presented can be implemented fundamentally also in combination with one another.

In an enhancement of the invention, the impedance measurement control device is designed for determining an electrode separation distance of the jaws during the sealing operation. This can be realized, for example, by analyzing the continuously measured impedance or by using a light-based or inductive or resistive distance sensor. Further process-relevant parameters can be derived from the electrode separation distance determined, such as the dimensions and the ectric properties of the vessel to be sealed and/or the desired ultimate geometry of the seal site.

In an enhancement of the invention, the HF jaw electrodes are designed to be thermally insulated. This contributes to further optimization of the energy efficiency of the device in that heat losses at the seal site are minimized.

In an enhancement of the invention, the device includes a cordless and/or handheld device body, which contains at least the clamp and the HF power supply circuit, preferably also the impedance measurement device. This contributes to a high user comfort of the device. The measures according to the invention and, in particular, the high energy efficiency achieved by the special impedance adjustment create the prerequisites for the cordless device design in contrast to vessel sealing devices of conventional design type, which necessitate a cord- or a cable-connected design.

In an enhancement of the invention, the device according to the invention comprises a rechargeable battery unit, such as, for example, a lithium rechargeable battery unit, as the electrical power source for the HF power supply circuit. Said lithium rechargeable battery unit can be accommodated in a handheld device body in a space-saving and weight-saving manner, for example, and likewise contributes to a high user comfort and ease of operation. In another configuration, the device according to the invention includes a charging station on which to set the device body and to charge the battery unit. This is of advantage for user comfort and ease of operation of the device, in particular in conjunction with a cordless design of the device body.

In a development of the invention, a thermal isolation is provided for the HF jaw electrodes. This contributes to achieving a high energy efficiency of the device.

In a development of the invention, the blood vessel sealing device is configured as a cordless sealing device. This provides a device with superior handling comfort.

Advantageous embodiments of the invention are illustrated in the drawings and will be described below. In the drawings:
- Fig. 1: a perspective view of a device body of a blood vessel sealing device,
- Fig. 2: a schematic block diagram of a blood vessel sealing device having an HF generator with variable resonant circuit capacitor capacitance,
- Fig. 3: a schematic block diagram, corresponding to Fig. 2, for a variant with variable resonant circuit coil inductance,
- Fig. 4: a diagram showing electrode separation distance vs. time for a typical sealing operation of a blood vessel sealing device,
- Fig. 5: a perspective view of a charging station for two device bodies of the type shown in Fig. 1,
- Fig. 6: a schematic block circuit diagram of an HF resonant circuit, which can be used in the device of Fig. 1, in a design with a jaw-external capacitor unit of variable capacitance at the start of a sealing operation,
- Fig. 7: the block circuit diagram of Fig. 6 at the end of a sealing operation,
- Fig. 8: a block circuit diagram corresponding to Fig. 6 for an embodiment variant with mechanical jaw coupling of a coil unit of variable inductance at the start of a sealing operation,
- Fig. 9: the block circuit diagram of Fig. 8 at the end of a sealing operation,
- Fig. 10: a block circuit diagram corresponding to Fig. 6 for a jaw-integrated capacitor unit of variable capacitance at the start of a sealing operation,
- Fig. 11: the block circuit diagram of Fig. 10 at the end of a sealing operation,
- Fig. 12: a block circuit diagram corresponding to Fig. 6 for an embodiment variant with an electronically controllable capacitance diode at the start of a sealing operation,
- Fig. 13: the block circuit diagram of Fig. 12 at the end of a sealing operation,
- Fig. 14: a block circuit diagram corresponding to Fig. 6 according to another embodiment of the invention at the start of a sealing operation,
- Fig. 15: the block circuit diagram of Fig. 14 at the end of a sealing operation,
- Fig. 16: a schematic sectional view of a clamp jaw part of a device like that of Fig. 1 with a jaw-integrated capacitor unit corresponding to Fig. 10 and 11 at the start of a sealing operation,
- Fig. 17: a view similar to Fig. 16 at the end of a sealing operation with part of a jaw actuating element additionally shown, and
- Fig. 18: a view corresponding to Fig. 17 for a modified embodiment.

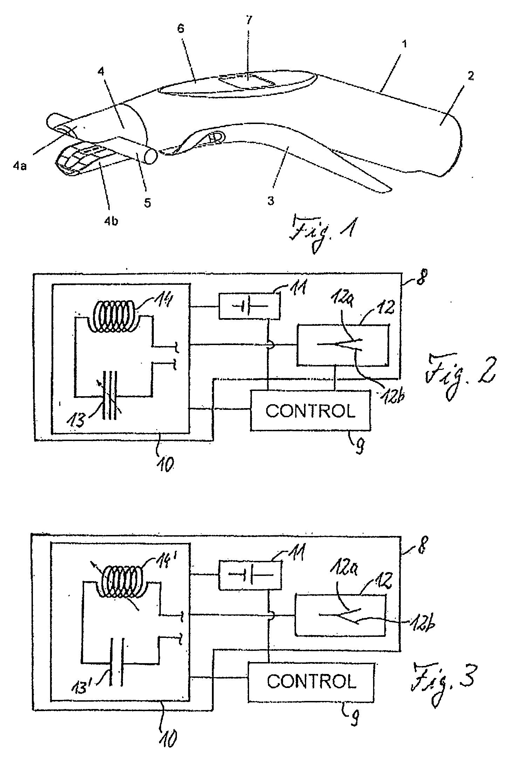

In an advantageous, exemplary embodiment type, the blood vessel sealing/sealing device according to the invention includes a cordless device body. Fig. 1 shows a cordless device body 1 of this type, which, in the implementation shown, can be held with one hand and operated by the user. For this purpose, the device body 1 has a back-side handle part 2 and a control lever 3 that serves as an electrical switch, which can be operated on the bottom side by the fingers of the hand holding the device body. On the front side, the device body 1 has a clamp 4, which comprises two jaws 4a, 4b, which can move relative to each other, for inserting and crimping a blood vessel 5, which is only partly indicated in Fig. 1. The blood vessel 5 can be, for example, an arterial vessel and the device can be designed correspondingly as a blood vessel sealing device. A display area 6 lies on the top side opposite the control lever 3 on the bottom side and is formed on the device body 1 between the back-side handle area 2 and the front-side clamp 4. The display area 6 comprises a display panel 7, on which desired information can be displayed optically.

In Fig. 2, an electrical high-frequency (HF) power supply circuit 8 and a device control 9 are shown schematically, such as can be used, for example, for a vessel sealing device with the cordless, handheld device body 1 of Fig. 1. The electrical HF power supply circuit 8 includes an HF generator 10, an electrical power source 11, and an electrode arrangement 12. The HF generator 10 is of a conventional design as such and includes a variable impedance HF resonant circuit with a capacitor unit 13 and a coil unit 14. In the example shown, the capacitor unit 13 is designed as one with variable, adjustable capacitor capacitance. For this purpose, the capacitor unit 13 can be designed in such a way, for example, that it has at least two capacitor electrodes, at least one of which can move with respect to the other in the direction of separation. Alternatively or additionally, it is possible to provide a dielectric element, which can move in the gap between two capacitor electrodes so as to change the capacitance.

The electrical power source 11 is implemented preferably as a rechargeable battery or accumulator unit; in particular, a lithium rechargeable battery unit or a lithium battery pack can be used for this, preferably one of lithium ion type, such as, in particular, a lithium polymer battery pack or a LiFePO₄ battery pack. Advantages of such electrical power sources are their relatively low weight for a relatively high storage capacity. In practical embodiments, it is thereby possible to achieve sealing capacities of more than 500 sealing operations before any recharging of the rechargeable battery unit 11 is required for a rechargeable battery weight of at most approximately 200 g, preferably at most 150 g.

The electrode arrangement 12 comprises two associated HF electrodes 12a, 12b, which are indicated only schematically in Fig. 2, one of which is arranged in each of the two jaws of the vessel sealing device, which can move relative to each other, for example, in the two jaws 4a, 4b of the clamp 4 in the device body 1 shown in Fig. 1. The HF generator 10 and the electrical power source 11 can also be accommodated in the device body 1; that is, the entire electric HF power supply circuit 8 is then situated in the device body 1. The jaws 4a, 4b, together with the HF electrodes 12a, 12b, are constructed preferably so as to achieve minimum energy/heat losses. For this purpose, they are provided with thermal insulation in a conventional way as such, which is not shown in detail and which ensures that heat losses arising from the seal site during the sealing operation are minimized.

The HF generator 10, supplied by the power source 11, supplies the HF power in a way known as such for the electrode arrangement 12 for sealing of a vessel placed between the HF electrodes 12a, 12b. The device control 9 controls and monitors the respective sealing operation, for which purpose it is suitably equipped. Besides conventional control means, which need not be addressed here in detail, the device control 9 according to the invention comprises, in particular, an impedance measurement and impedance control device for continuous measurement of the impedance and for maintaining constant the impedance of the HF power supply circuit 8 in the course of the respective sealing operation. The device control 9 is equipped with suitable hardware and software components, as are known to the person skilled in the art who understands the functionalities of the device control 9 explained here. In particular, for this purpose, the device control 9 contains suitable computing components, such as, for example, a conventional microcontroller. In the embodiments with the cordless and handheld device body 1 of Fig. 1, the device control 9 can be arranged together with all of its components or together with only a part of its components, as needed, in the device body 1. Further alternatively, the device control 9 can be arranged completely outside of the device body 1 and can be connected so as to be in communication with the HF power supply circuit 8, accommodated in the device body 1, via a suitable, conventional, wireless interface. For example, this can be a Bluetooth interface.

For carrying out a sealing operation using the device corresponding to Figs. 1 and 2, for example, the vessel 5 is placed between the jaws 4a, 4b and thus between the HF electrodes 12a, 12b and then the jaws 4a, 4b are moved toward each other by operating the control lever 3, as a result of which the vessel 5 is crimped. At the same time, the HF power supply circuit 8 is activated and supplies the high-frequency energy, which is required for melting the vessel material, to the sealed vessel 5 at the crimp or pinched-off site between the jaws 4a, 4b via the HF electrodes 12a, 12b. Owing to the closing movement of the jaws 4a, 4b and thus of the HF electrodes 12a, 12b toward each other, the electrode separation distance of the HF electrodes 12a, 12b is correspondingly changed, as a result of which the impedance of the HF power supply circuit 8 would be changed if no counteractions were taken. The deformation and heating of the vessel material at the crimp site between the HF electrodes 12a, 12b can also contribute to this. Such a change in impedance would result in a marked decrease in the energy efficiency of the device. Although this could be counteracted by an appropriate change in the frequency of the high-frequency radiation provided for the sealing operation, this could lead to undesired secondary effects.

The invention therefore provides for other counteractions, namely, keeping the impedance of the HF power supply circuit 8 constant throughout the course of the sealing operation. For this purpose, the impedance measurement and control device of the device control 9 continuously registers the current value or actual value of the impedance throughout the course of the respective sealing operation and provides for any required adjustment or tracking by adjusting or tracking the variable capacitor capacitance of the capacitor unit 13. For this purpose, the device control 9 controls the movement of the capacitor electrode or of the dielectric element in such a way that the impedance of the power supply circuit 8 is maintained constant at each point in time during the sealing operation, which obviously entails the possibility of maintaining the impedance only essentially constant and allowing for minor temporary deviations. Any measurement devices known for the purpose of complex impedance measurement can be used for impedance measurement.

The impedance can be tracked preferably by mechanical movement of elements that influence the impedance inductively, capacitively, or resistively. The impedance can be tracked preferably by way of electronic components, such as, for example, capacitance diodes, without any mechanical movement. Depending on need and applied case, the device control 9 can derive further parameters and information of interest from the measurement of the impedance and the change in time thereof, such as the electrode separation distance of the HF electrodes 12a, 12b, the material of the vessel, the thickness of the vessel prior to and/or during the sealing operation, and/or the detection as to whether a vessel has been placed between the HF electrodes 12a, 12b. Tissue of vessels are consisting of variable amounts of water, electrolytes, proteins and lipids, and for a given HF energy, heat at different rates, so that from the change in time of the impedance during the sealing operation and, in particular, in an early phase thereof, the device control 9 can determine individual properties of a given vessel. In the implementation using the device body of Fig. 1, the device control 9 can display desired information on the display unit 7, such as, for example, the state of charge of the rechargeable battery unit 11 and/or the number of sealing operations still presumably possible for the current state of charge.

For example, a shift in the resonance frequency owing to the change in impedancecan lead to considerable loss of efficiency. An impedance mismatch between parts of the HF circuit can result in reflection of waves, resulting also in a loss of efficiency By keeping the impedance constant in accordance with the invention, it is possible to maintain the high frequency essentially at the desired resonance frequency throughout the entire course of the sealing operation. Correspondingly, the energy efficiency can be optimally maintained. The tracking effected by the device control 9 and maintaining the impedance of the HF power supply circuit 8 constant throughout the entire course of the sealing operation allow for a good and unvarying quality of the seal with minimum energy consumption and with optimized sealing time.

A suitable detection of the end point of the sealing operation can also contribute for this purpose. The device control 9 can determine the actual properties of the crimped blood vessel at the seal site from, for example, the continuously measured impedance of the HF power supply circuit 8 or a continuous direct measurement of the electrode separation distance or the distance of the jaws 4a, 4b from each other. For direct measurement of the electrode separation distance or jaw separation distance, the device control 9 can be associated with a corresponding conventional distance sensor. Such a distance sensor of conventional type can, for example, be light-based or it can be of an inductive or resistive sensor type.

Fig. 3 illustrates a variant of the blood vessel sealing device according to Fig. 2, for which identical reference numbers are used for identical and functionally equivalent components and insofar reference can be made to the above description in regard to Fig. 2. In the device variant of Fig. 3, the adjustment or tracking of the impedance of the HF power supply circuit 8 is provided for the purpose of maintaining a constant impedance by appropriate or tracking change/varying of the inductance of an appropriately modified coil unit 14' of the HF generator 10, instead of the coil unit 14, with invariable inductance in the example of Fig. 2. In this case, it is possible, as shown, to employ an appropriately modified capacitor unit 13' with constant capacitor capacitance. The variable inductance can be provided by the coil unit 14' in that, for example, the latter has a movable inductance-altering element, preferably a ferrite element, as is known as such, with the device control 9 controlling the movement of the ferrite element in such a way that the inductance of the HF power supply circuit 8 is maintained constant.

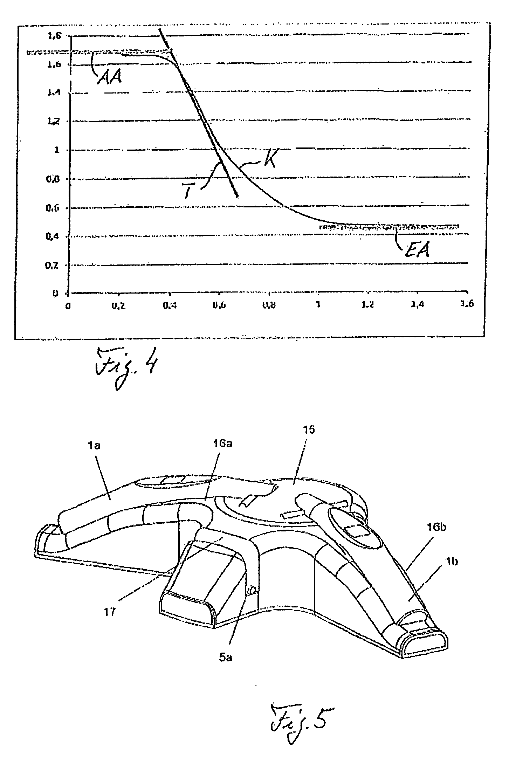

Otherwise, the same characteristics and advantages apply to the device according to Fig. 3 as those explained above for the device according to Fig. 2. In another alternative device variant, both the capacitor capacitance and the coil inductance of the HF generator 10 are varied in order to ensure that the impedance of the HF power supply circuit 8 remains constant. For this purpose, the HF generator 10 can be constructed together with the capacitor unit 13 of variable capacitor capacitance of Fig. 2 and together with the coil unit 14' of variable inductance of Fig. 3. Fig. 4 illustrates a typical characteristic K for the electrode separation distance of the HF electrodes 12a, 12b as a function of time for a typical sealing operation. The device control 9 is equipped, as explained, for recording the curve of the characteristic K. Prior to and at the start of the sealing operation, the recorded electrode separation distance represents the outer diameter of the inserted blood vessel, readable for the device control 9, on the basis of an associated horizontal initial asymptote AA of the characteristic curve K. When the sealing operation starts, the jaws of the clamp of the blood vessel sealing device and thus of the HF electrodes approach each other, with the change in time of the electrode separation distance for a given HF power being determined by the rate of heating or rate of shrinking of the blood vessel material. In accordance therewith, the device control 9 can draw a conclusion about the properties of the blood vessel, from the slope of a tangent T to the characteristic K in a first vessel heating portion.

From the blood vessel parameters thus determined the device control 9 can then determine the optimum material thickness of the seal to be produced. The device control 9 can utilize this in order to suitably adjust or specify in advance the HF heating power and the end point of the sealing operation. Correspondingly, the characteristic K of the time course of the electrode separation distance then transitions toward the end of the sealing operation to a horizontal end asymptote EA, the associated electrode separation distance value of which represents the desired target thickness of the seal of the given blood vessel.

Fig. 5 illustrates a charging station 15, which has two holders 16a, 16b for holding two device bodies 1 a, 1 b corresponding to the device body 1 of Fig. 1. When the respective device body1a, 1 b is placed in one of the holders 16a, 16b of the charging station 15, the rechargeable battery unit 11 situated in it can be recharged electrically by the charging station 15. At the same time, the charging station 15 serves as a place to rest the device bodies 1a, 1 b. The charging station 15 serves in this way as a docking station for the device bodies 1 a, 1 b. In the process, charging times of less than one hour can be realized for a lithium rechargeable battery unit, for example. In the example shown, the charging station 15 has, in addition, a slot holder 17, in which a vessel 5a can be accommodated.

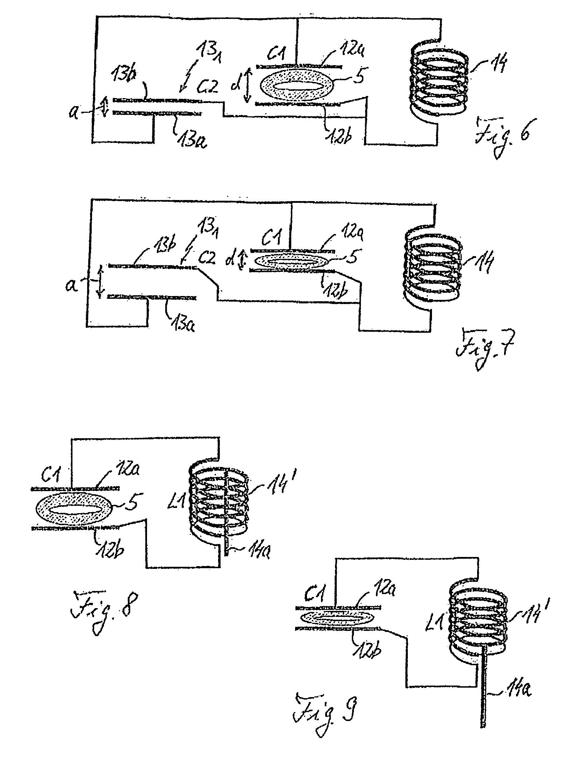

In Figs. 6 to 18, specific embodiment variants for maintaining constant or tracking the impedance of the HF power supply circuit are illustrated schematically for the blood vessel sealing/sealing device according to the invention with the components of interest for this purpose, in each case in the state prior to the start of and at the end of a sealing operation. This is represented by the HF electrodes 12a, 12b and the blood vessel 5 clamped between them, where the HF electrodes 12a, 12b arranged in the jaws of the device move toward each other during the sealing operation and, as a result, their mutual separation distance d decreases and, in consequence thereof, their electrical capacitance C1, which influences the behavior of the HF resonant circuit, increases.

In the exemplary embodiment of Figs. 6 and 7, besides coil unit 14, a capacitor unit 13₁ of variable capacitor capacitance C2 is looped in parallel to the capacitance formed by the HF electrodes 12a, 12b; that is, one of two respective electrodes 13a, 13b of this capacitor unit 13₁ is electrically coupled so as to lie at the same potential with one of the two HF electrodes 12a, 12b. In addition, for the purpose of variably changing their separation distance and thus their capacitor capacitance, the two capacitor electrodes 13a, 13b are arranged so as to be movable in relation to each other. This can be accomplished, for example, in that one of the two capacitor electrodes 13a, 13b, for example, the electrode 13b, is arranged on a device component that moves together with the jaw movement of the device during the sealing operation, while the other capacitor electrode 13a is arranged on a device component that does not move together with the jaw movement. Accordingly, the movable capacitor electrode 13b can be arranged on a control lever for the jaw movement, for example, and the other capacitor electrode 13a can be arranged on an opposite-lying housing part of the device.

As illustrated in Figs. 6 and 7, the capacitor electrodes 13a, 13b are arranged in such a manner that they increase their mutual separation distance a when the separation distance d of the HF electrodes 12a, 12b decreases during the sealing operation. In this case, the measure of the change in separation distance of the capacitor electrodes 13a, 13b is chosen in such a way that the capacitance C2 of the capacitor unit 13₁, which decreases owing to the increase in separation distance, compensates for the increase in the capacitor capacitance C1 thereof effected by the decrease in the separation distance of the HF electrodes 12a, 12b, so that the total capacitance C1+C2 of the HF resonant circuit remains constant during the sealing operation. In an advantageous embodiment, this can be accomplished by a corresponding mechanical coupling of the movable capacitor electrode 13b to the movement of one of the jaws and thus the HF electrode 12b thereof. Alternatively, the tracking of the separation distance can be provided electronically for the capacitor electrodes 13a, 13b, depending on the recorded jaw movement or the change in impedance resulting from it. In the case of mechanical coupling, it is possible, as needed, to dispense with the control shown in Figs. 2 and 3 or the control can be implemented in a correspondingly simplified manner.

Figs. 8 and 9 illustrate an embodiment variant in which the change in the capacitance C1 of the HF electrodes 12a, 12b arising during the sealing operation is compensated for by a tracking of the inductance L1 of the coil unit 14' with variable inductance, for which purpose the coil unit 14' has a movable inductance-altering element in the form of a ferrite element 14a that can move axially in the coil. The axial inward movement of the ferrite core 14a into the coil and its outward movement out of the latter occurs, in turn, in a manner that depends on the jaw movement during the sealing operation and thus the capacitance-altering movement of the HF electrodes 12a, 12b in such a way that the total impedance of the HF resonant circuit remains essentially constant. For this purpose, for example, the ferrite core 14a is moved further out of the coil with closing jaw movement, as is illustrated in Figs. 8 and 9. The movement of the ferrite core 14a, which depends on the jaw closing movement, as explained above in regard to Figs. 6 and 7, can be provided alternatively by way of an electronic control or by way of a mechanical coupling of the movement of the ferrite core 14a to one of the jaws, for example, to the jaw that contains the HF electrode 12b.

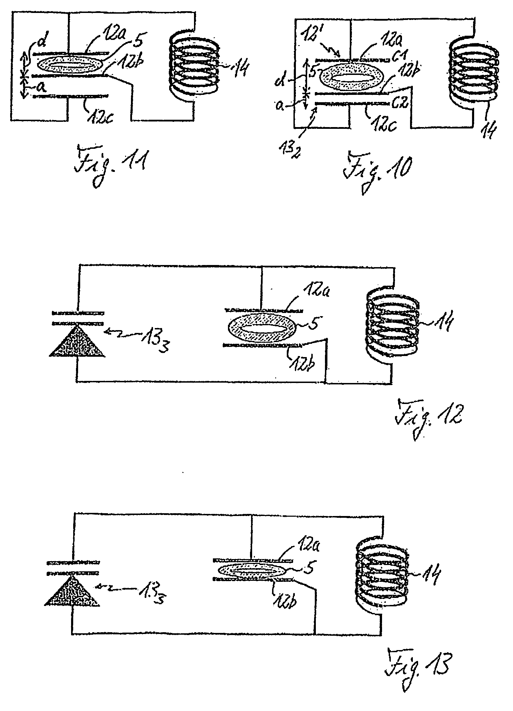

Figs. 10 and 11 illustrate a modification of the exemplary embodiment of Figs. 6 and 7 in that a capacitor unit 13₂ of variable capacitance is integrated in the jaws of the device. For this purpose, the clamp is designed with three jaws of which a first outer jaw and an intermediate jaw bear the two HF electrodes 12a, 12b, while a second outer jaw carries the capacitor electrode 12c of this capacitor electrode 13₂. Its other capacitor electrode is provided by the HF electrode 12b of the intermediate jaw. The outer capacitor electrode 12c is electrically coupled to the outer HF electrode 12a so as to lie at the same potential. In this way, as in the example of Figs. 6 and 7, there exists a parallel connection of the capacitance C1 of the HF electrodes 12a, 12b to the capacitance C2 of the capacitor unit 13₂. The vessel 5 to be sealed lies between the two jaws bearing the HF electrodes 12a, 12b.

Accordingly, the two HF electrodes 12a, 12b move toward each other, in turn, during a sealing operation, as a result of which the capacitance C1 thereof increases, while, however, at the same time, the separation distance a between the two capacitor electrodes 12b, 12c increases, so that the capacitor capacitance C2 thereof decreases and, as a result, the total capacitance C1+C2, in turn, remains essentially constant. In this embodiment variant, the movement of the jaws thus itself changes the separation distance a of the variable capacitance 13₂ in the sense of maintaining the impedance of the HF resonant circuit constant, so that, in this example, a corresponding additional control is not absolutely necessary.

In an embodiment variant illustrated in Figs. 12 and 13, an electronically controllable capacitance diode 13₃ serves as capacitor unit with variable capacitance. The diagram in Fig. 13 shows a diagram of the principle, without consideration of the maximum voltages. In another embodiment, the tunable resonant circuit can be separated inductively or capacitively from the load resonant circuit. The capacitance thereof can be varied by a variable direct-current voltage that overlaps the resonant voltage of the HF resonant circuit, as is known as such to the person skilled in the art, and therefore needs no further explanation here. A control or regulation, which is not shown, records the actual impedance of the HF resonant circuit or a parameter responsible for it, such as the separation distance d of the HF electrodes 12a, 12b, and controls the capacitor diode 13₃ with the direct-current voltage required for maintaining the impedance of the HF resonant circuit constant by means of a corresponding change in capacitance. Here also, the capacitance of the capacitance diode 13₃ is once again looped in electrically parallel to the capacitance of the HF electrodes 12a, 12b in the HF resonant circuit. Moreover, the explanations made in regard to the exemplary embodiments of Figs. 6 and 7 and of Figs. 10 and 11 above apply to these embodiment variants in an identical way.

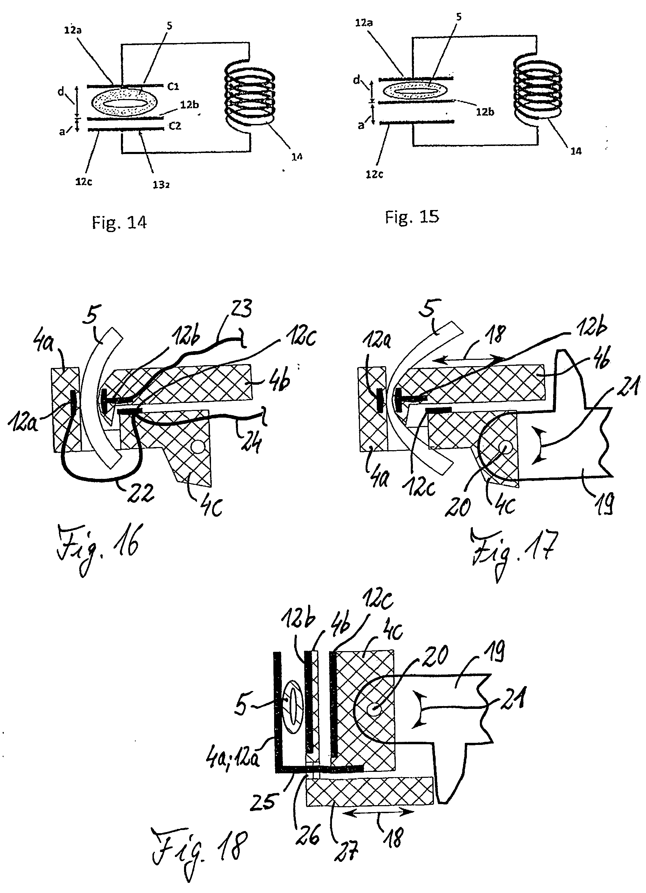

Figures 14 and 15 illustrate another modification of the exemplary embodiment of Figs. 6 and 7 to the effect that a capacitor unit 13₂ of variable capacitance is integrated in the jaws of the device. This modification is generally similar to that shown in Figs. 10 and 11. In this case, identical reference numbers identify components that are identical or similar to those in Fig. 10 or 11, the description of which will not be repeated. The modification of the device according to the invention shown in Figs. 14 and 15 differs from the embodiment shown in Figs. 10 and 11 in that the capacitance C1 formed by the RF electrodes 12a, 12b is connected in series with the capacitance C2 formed by the RF electrodes 12b, 12c. The function and course of the sealing operation are identical to the operation described in regard to Figs. 10 and 11. In this example, only the intermediate electrode 12b moves, with the total capacitance of the sealing jaw remaining constant. In this modification, Fig. 14 shows the state prior to the start of the sealing operation and Fig. 15 shows the state after conclusion of the sealing operation.

Figs. 16 and 17 illustrate an embodiment incorporating the principle of integrating fixed and movable capacitor electrodes of the capacitor unit of the variable impedance HF resonant circuit in a jaw part of the blood vessel sealing device, more specifically a jaw and capacitor electrode arrangement according to the principles of the embodiment illustrated in Figs. 10 and 11 explained above. For easy understanding the same reference numbers are thus used as in Figs. 10 and 11.

In the arrangement of Figs. 16 und 17 the HF electrode 12a is embedded in material of the first jaw 4a, the capacitor electrode 12c is mounted at a fixed clamp body part 4c, and the movable intermediate electrode 12b is embedded in a material of the other jaw 4b. The two jaws 4a, 4b and the adjacent clamp part 4c may be fabricated from acetal plastic material. The jaw 4b and thus its embedded electrode 12b is movable relative to the jaw 4a and the fixed clamp part 4c as illustrated by arrow 18 so as to vary the distance between the two jaws 4a and 4b for clamping and sealing blood vessel 5 inserted between the jaws 4a, 4b. When moving jaw 4b towards jaw 4a to conduct the sealing operation for vessel 5, the distance between the movable electrode 12b and the fixed electrode 12a decreases. At the same time the effective distance between movable electrode 12b and fixed electrode 12c increases, that means the corresponding opposing areas of the two electrodes 12b, 12c are reduced so that the capacitance provided by the electrodes 12b and 12c is reduced. Fig. 17 shows the clamp jaws 4a, 4b at the end of the sealing operation, for which operation HF is applied through the HF jaw electrodes 12a, 12b. The increased jaw electrode capacitance is compensated by the decreased capacitance of the capacitor formed by the two electrodes 12b and 12c. By using the acetal plastic material a desired thermal isolation of the jaws 4a, 4b can be accomplished.

The movement of the jaw 4b relative to the jaw 4a and the fixed clamp part 4c is accomplished by the use of an actuating element 19 of the device. The actuating element 19 is pivotably mounted to the fixed clamp body part 4c at pivot axis 20, as illustrated by arrow 21. In the embodiment according to figure 1, the actuating element 19 may be the control lever 3 of device body 1 or may be suitably coupled to said control lever 3.

In the embodiment of Figs. 16 and 17, the electrodes 12a and 12c are of plate-like shape and arranged in orthogonal planes. The intermediate electrode 12b is adapted to this by having a T-like cross-section form with its head cooperating with the electrode 12a, while with its foot part cooperating with the electrode 12c. According to the electrical arrangement of Figs. 10 and 11, the electrodes 12a and 12c are short-circuited to remain on a same voltage level by an electric connection wire 22. The electrodes 12b and 12c are connected to coil 14, not shown in Figs. 16 and 17, through corresponding connection wires 23, 24.

Fig. 18 shows a modified arrangement of the integration of the capacitor electrodes of a variable capacitor of the capacitor unit similar to the embodiment of Figs. 16 and 17. Again, same reference numbers are used for identical or functionally equivalent elements to facilitate understanding. The embodiment of Fig. 18 can be used e.g. to realize an arrangement like that of Figs. 14 and 15.

In the embodiment of Fig. 18 the fixed jaw electrode 12a, the fixed capacitor electrode 12c, and the intermediate, movable, combined jaw and capacitor electrode 12b are all formed as effective plate-like electrodes arranged parallel to each other. The jaw electrode 12a in this example forms the jaw 4a and to this end is fixed at the clamp body part 4c via a fixing leg 25. The other jaw 4b is of a plate-like shape and supports the intermediate, movable electrode 12b. In this case the jaw 4b is movably guided along the leg 25 which extends through a corresponding opening 26. In addition, the jaw 4b is provided with a base part 27 as an interface to the actuating element 19 for moving the jaw 4b relative to the jaw 4a and the fixed clamp part 4c.

The electrodes 12a, 12b, 12c are provided with proper electrical connections not shown in Fig. 18, so as to realize the desired circuitry, e.g. the one according to Figs. 14 and 15, or alternatively the one of Figs. 10 and 11.

In embodiments of the invention that are not shown, the vessel sealing device is designed as a stationary stand-alone device. In other alternative embodiments of the invention, the vessel sealing device has a handheld device body, which corresponds for the most part to that of Fig. 1, but is designed in a cable-connected manner. In this case, the device components accommodated in the device body are connected via a corresponding cable connector to the other components of the vessel sealing device arranged outside of the device body. Depending on the case of application, it is possible, for example, to arrange the entire device control or a part thereof and/or the electrical power source outside of the device body.

As the above-mentioned exemplary embodiments make clear, the invention provides an advantageous vessel sealing device, which can be designed, as needed, as a mobile device with low weight and a cordless device body, the vessel sealing device according to the invention making possible a high energy efficiency and process accuracy for the sealing operation. In particular, continuously maintaining the impedance constant for the HF energy supplied for the sealing operation throughout the entire course of the sealing operation contributes to this result. A rechargeable battery unit of low weight can be utilized for the device according to the invention. Changes in frequency of the high-frequency radiation during the sealing operation can be avoided.

## Claims

**1.** A blood vessel sealing device, in particular a vessel sealing device, comprising
- a clamp (4) which contains a pair of jaws (4a, 4b) that can move relative to each other for inserting and crimping a blood vessel, said jaws containing high-frequency (HF) jaw electrodes (12a, 12b),
- an electrical HF power supply circuit (8), comprising an HF generator (10), which includes a variable impedance HF resonant circuit with a capacitor unit (13, 13') and a coil unit (14, 14'), and comprising the jaw electrodes, wherein the inductance of the coil unit and/or the ohmic resistance of the HF resonant circuit is/are variably adjustable and/or wherein the capacitance of the capacitor unit is variably adjustable and the capacitor unit comprises an electrically controllable capacitance diode (13₃) or at least one movable capacitance-altering capacitor electrode (13a, 13b; 12b,12c) arranged in the clamp, and
- an impedance control device (9) configured for acting towards maintaining an impedance of the HF power supply circuit constant during a respective sealing operation by correspondingly controlling the variable impedance HF resonant circuit.

**2.** The blood vessel sealing device according to claim 1, wherein the capacitor unit comprises a movable capacitance-altering dielectric element and/or wherein the coil unit has a movable inductance-altering element, preferably a ferrite element.

**3.** The blood vessel sealing device according to claim 1 or 2, wherein the movable capacitance-altering capacitor electrode is arranged in the clamp in such a way that a closing movement of the jaw electrodes (12a, 12b) is compensated for by a movement of capacitor electrodes of the capacitor unit connected in series or in parallel to the jaw electrodes.

**4.** The blood vessel sealing device according to any one of claims 1 to 3, wherein the movable capacitance-altering capacitor electrode is mechanically coupled to one of the jaws containing the jaw electrodes.

**5.** The blood vessel sealing device according to any one of claims 1 to 3, wherein the movable capacitance-altering capacitor electrode is mechanically coupled to the jaw electrodes by a heat-deforming element.

**5.** The blood vessel sealing device according to any one of claims 1 to 4, wherein a same electrode (12b) forms one of the capacitor electrodes (12b, 12c) of the capacitor unit (13₂) and one of the jaw electrodes (12a, 12b).

**6.** The blood vessel sealing device according to claim 5, wherein said same electrode forms an intermediate electrode positioned between two outer electrodes (12a, 12c) forming a counter electrode (12c) of the capacitor unit and the other jaw electrode (12a), respectively.

**7.** The blood vessel sealing device according to claim 6, wherein said other jaw electrode (12a) and said counter electrode (12c) are coupled electrically to a same potential so that a capacitance (C1) of the jaw electrodes (12a, 12b) and a capacitance of the capacitor unit (13₂) are connected in parallel.

**8.** The blood vessel sealing device according to claim 6, wherein the intermediate electrode (12b), the other jaw electrode (12a), and the counter electrode (12c) are arranged so that a capacitance (C1) of the jaw electrodes (12a, 12b) and a capacitance of the capacitor unit (13₂) are connected in series.

**9.** The blood vessel sealing device according to any one of claims 6 to 8, wherein the intermediate electrode (12b) forms the movable capacitance-altering capacitor electrode of the capacitor unit.

**10.** The blood vessel sealing device according to any one of the preceding claims, wherein the impedance control device is designed for determining an electrode separation distance of the jaws prior to and/or during the respective sealing operation.

**11.** The blood vessel sealing device according to any one of the preceding claims, wherein the jaw electrodes are designed to be thermally insulated.

**12.** The blood vessel sealing device according to any one of the preceding claims, further comprising a cordless and/or handheld device body (1) which contains at least the clamp and the electrical HF power supply circuit.

**13.** The blood vessel sealing device according to any one of the preceding claims, further comprising a rechargeable battery unit (11) as electrical power source for the electrical HF power supply circuit.

**14.** The blood vessel sealing device according to claim 13, further comprising a charging station for resting the device body and for charging the battery unit.

**15.** The blood vessel sealing device according to any one of the preceding claims, wherein a thermal isolation is provided for the HF jaw electrodes and/or the blood vessel sealing device is configured as a cordless sealing device.
